# EUROPÄISCHE PATENTSCHRIFT

(11) **EP 1 641 731 B1**
(45) Veröffentlichungstag und Bekanntmachung des Hinweises auf die Patenterteilung: **20.02.2008**
(21) Anmeldenummer: 04731379.6
(22) Anmeldetag: 06.05.2004
(51) Int. Cl.: C07C 1/20, C07C 11/02, C07C 7/00

(54) **VERFAHREN ZUR HERSTELLUNG VON 1-OCTEN AUS CRACK-C4**
METHOD FOR PRODUCING 1-OCTENE FROM CRACK-C4
PROCEDE DE PRODUCTION DE 1-OCTENE A PARTIR DE CRACK-C4

(30) Priorität: 27.06.2003 DE 10329042
(43) Veröffentlichungstag der Anmeldung: 05.04.2006
(73) Patentinhaber: Oxeno Olefinchemie GmbH, 45772 Marl (DE)
(72) Erfinder: KRISSMANN, Jörg, 63801 Kleinostheim (DE); RÖTTGER, Dirk, 45657 Recklinghausen (DE); BORGMANN, Cornelia, 45657 Recklinghausen (DE); KÄMPER, Kerstin, 45731 Waltrop (DE); NIERLICH, Franz, 45768 Marl (DE); KAIZIK, Alfred, 45772 Marl (DE); KNIPPENBERG, Udo, 45772 Marl (DE); MALZKORN, Rainer, Mobile, Alabama 36695 (DE)
(74) Vertreter: Hirsch, Hans-Ludwig
(86) Internationale Anmeldenummer: PCT/EP2004/050722
(87) Internationale Veröffentlichungsnummer: WO 2005/000772

(56) Entgegenhaltungen:
- WO-A-92/10450
- DE-A- 10 149 348

## Beschreibung

Die Erfindung betrifft ein Verfahren zur Herstellung von 1-Octen aus Crack-C₄ durch Telomerisation des im Crack-C₄ enthaltenen 1,3-Butadien mit Methanol in Gegenwart eines Katalysators, Hydrierung des so erhaltenen Telomers, Spaltung des hydrierten Telomers und Aufarbeitung des so erhaltenen Spaltprodukts zum reinen 1-Octen.

1-Octen wird in großen Mengen in der Produktion verschiedener chemischer Produkte eingesetzt. Beispielsweise werden oberflächenaktive Stoffe, Weichmacher, Schmierstoffe und Polymere aus 1-Octen hergestellt Ein großes Einsatzgebiet ist weiterhin die Verwendung als Comonomer in Polymeren, insbesondere in Polyethylen.
Nahezu alle zur Zeit kommerziell genutzten Verfahren zur Produktion von 1-Octen basieren auf dem Rohstoff Ethen. Ethen wird oligomerisiert und man erhält ein Produktspektrum von α-Olefinen als Hauptprodukte. Bei passender Wahl von Katalysator und Prozessbedingungen kann die Menge an 1-Octen im Produkt optimiert werden und liegt dann bei ca. 25 %. Neben diesen Verfahren, mit denen die Hauptmenge an 1-Octen produziert wird, hat die Isolierung des 1-Octens aus dem Produktspektrum der Fischer-Tropsch-Reaktion eine gewisse Bedeutung erlangt

In der Literatur sind neben den auf Ethen basierenden Prozessen auch Verfahren bekannt, die 1,3-Butadien als Rohstoff einsetzen. 1-Octen ist aber nicht direkt, beispielsweise über eine Dimerisierung, aus Butadien erhältlich, sondern wird nach mehreren Prozessschritten erhalten. So beschreibt die Patentanmeldung WO 92/10450 ein Verfahren, bei dem 1,3-Butadien vorzugsweise mit Methanol oder Ethanol zu einem 2,7-Octadienylether umgesetzt wird, der nach Hydrierung zum Octylether zum 1-Octen gespalten wird. In EP-A-0 440 995 wird ein analoger Weg beschritten, die Umsetzung erfolgt im ersten Schritt aber mit einer Carbonsäure. Gemeinsam ist den Verfahren der erste Prozessschritt, den man allgemein als Telomerisation bezeichnet Bei der Telomerisation wird allgemein ein Telogen (in EP-A-0 440 995 die Carbonsäure) mit einem Taxogen (1,3-Butadien, 2 Äquivalente) zu einem Telomer umgesetzt.

Neuere Verfahrensvarianten sind beispielsweise in DE 10 10 5751, DE 10 12 8144, DE 1014 9348, DE 1014 9347 und DE 10 22 9290 beschrieben.

Diese Verfahren wenden die o. g. Schritte der Telomerisation, Hydrierung und anschließende Spaltung an und erzeugen neben dem gewünschten Zielprodukt 1-Octen Nebenprodukte, die aus dem Zielprodukt abgetrennt werden müssen. Da 1-Octen häufig als Comonomer verwendet wird, ist die Herstellung von hochreinem 1-Octen wünschenswert. Die vorliegende Erfindung löst diese Aufgabe.

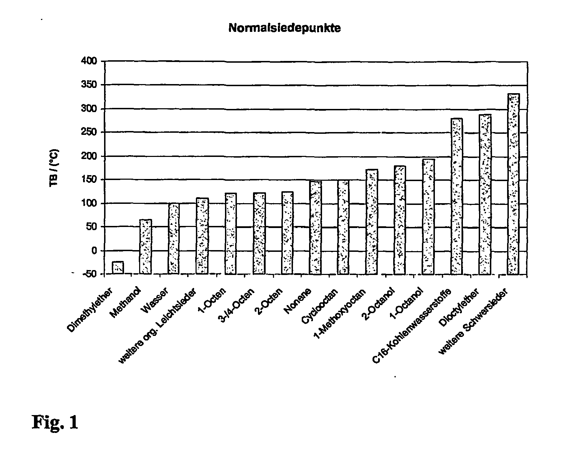

Zur Verdeutlichung des nicht trivialen Trennproblems zeigt die folgende Tabelle 1 die typische Zusammensetzung eines gemäß den o. g. Verfahren erhaltenen Spaltprodukts; Fig. 1 zeigt die zugehörigen Siedepunkte. Es ist nun leicht ersichtlich, dass durch einfache Destillation des Spaltprodukts eine Abtrennung des 1-Octens in den gewünschten Reinheitsgraden nicht möglich ist

**Tabelle 1: Beispielhafte Zusammensetzung eines Spaltproduktes**

| Komponente | Anteil Gew.-% |
|---|---|
| Dimethylether | 5,90 |
| Methanol | 1,50 |
| Wasser | 2,30 |
| KW C1-C7 | 0,02 |
| 1-Octen | 33,90 |
| 2-Octene | 1,70 |
| 3/4-Octene | 0,63 |
| 1-Octanal | 2,76 |
| 2-Octanol | 0,26 |
| 2-Octanon | 0,15 |
| Andere C8 KW | 0,24 |
| KW C9 | 1,36 |
| KW >C9 | 0,53 |
| 1-Methoxyoctan | 46,00 |
| Dioctylether | 1,70 |
| Rest | 1,05 |

Es wurde nun überraschender Weise gefunden, dass trotz dieser komplexen Zusammensetzung die Herstellung von 1-Octen aus Crack-C₄ in ausreichender Reinheit durch ein besonderes Destillationsverfahren, ggf. mit einer vorgeschalteten Wasserwäsche, möglich ist

Gegenstand der vorliegenden Erfindung ist daher ein Verfahren zur Herstellung von 1-Octen durch
a) katalytisches Umsetzen eines Butadien-haltigen Stroms mit Methanol unter Erhalt eines mindestens 1-Methoxy-2,7-Octadien-haltigen Stroms
b) katalytische Hydrierung des 1-Methoxy-2,7-Octadien-haltigen Stroms zu einem mindestens 1-Methoxyoctan-haltigen Strom
c) katalytische Spaltung mindestens eines Teils des 1-Methoxyoctans zu einem Spaltprodukt, enthaltend mindestens Wasser und 1-Octen,
   dadurch gekennzeichnet,
   dass
d) das Spaltprodukt aus c) destillativ in eine dampfförmige Leichtsiederfiaktion, enthaltend mindestens 1-Octen und Wasser und eine flüssige Schwersiederfraktion, enthaltend mindestens 1-Octen und 1-Methoxyoctan getrennt,
e) die Leichtsiederfraktion ganz oder teilweise kondensiert und in eine wässrige und eine 1-Octen enthaltende, unpolare Phase getrennt,
f) die unpolare Phase aus e) in die Stufe d) rückgeführt und
g) die Schwersiederfraktion aus d) in eine 1-Octen-haltige und eine 1-Methoxyoctan-haltige Fraktion getrennt wird.

Die Verfahrensschritte a) bis c) des erfindungsgemäßen Verfahrens unterscheiden sich nicht vom Stand der Technik und können z. B. in den o. g. Patentdokumenten, insbesondere in DE 10 10 5751, DE 10 12 8144, DE 10 14 9348, DE 10 14 9347 und DE 10 22 9290 nachgelesen werden. Auf diese Verfahren wird hier ausdrücklich Bezug genommen.

In Verfahrensschritt a) des erfindungsgemäßen Verfahrens, der die Telomerisation umfasst, können Butadien-haltige Ströme, z. B. sogenannte Crack-C₄-Ströme eingesetzt werden. Typische Butadienkonzentrationen in diesen Strömen liegen bei 20 bis 70 % 1,3-Butadien. Die übrigen Komponenten n-Butan, Iso-Butan, 1-Buten, 2-Buten und Iso-Buten stören die Umsetzung im Telomerisationsschritt nicht oder nicht wesentlich. Andere Diene, wie z. B. Alle,ne, oder Acetylene, insbesondere Vinylacetylen werden jedoch vorteilhaft durch Destillation, Extraktion oder selektive Hydrierung entfernt

Als bevorzugte Telomerisationskatalysatoren werden Nickel-, Rhodium-, Palladium- oder Platin-Katalysatoren, beispielsweise solche mit phosphorhaltigen Liganden wie Phosphine (z. B. Triphenylphosphin), Phosphite (z. B. Trimethylphosphit), Phosphonite oder Phosphinite (z. B. Diphenylphenoxyphosphin) eingesetzt. Bevorzugt ist der Einsatz dieser Katalysatoren mit Carbenliganden. Die Verwendung einer Base wie z. B. Metallhydroxide, Alkoholate, Phenolate, oder eines Lösungsmittels wie z. B. inerte aliphatische Kohlenwasserstoffe in diesem Verfahrensschritt ist optional.

Die Telomerisationsreaktion wird bevorzugt zwischen 10 °C und 200 °C und einem Reaktionsdruck von 1 bis 300 bar durchgeführt.

Als Telogen wird im erfindungsgemäßen Verfahren ausschließlich Methanol verwendet, wobei pro Mol Methanol zwischen 0,1 Mol bis 4 Mol 1,3-Butadien eingesetzt werden können.

Das nach Schritt a) erhaltene 1-Methoxy-2,7-Octadien wird in Schritt b) hydriert.

Die Hydrierung kann als Flüssig- und/oder Gasphasenhydrierung oder in Kombination dieser Techniken erfolgen und sie kann in einem oder mehreren Schritten erfolgen wie beispielsweise in einer Vor- und einer Endhydrierung.

Die Hydrierung kann kontinuierlich oder diskontinuierlich durchgeführt werden. Als Reaktoren können die bekannten Standards für Hydrierungen eingesetzt werden, beispielsweise Tricklebed-Reaktoren. Die bei der Reaktion entstehende Reaktionswärme wird nach bekannten Verfahren abgeführt, beispielsweise durch interne oder externe Kühler. Konkret kann dies den Einsatz von Rohrbündelreaktoren, Kühlfingern, Kühlschlangen oder -platten oder die Kühlung eines Rückführungsstroms (Reaktoren mit Kreislauf, Recycling), bedeuten.

Die Hydrierung wird katalysiert durchgeführt. Es können dabei sowohl homogene als auch heterogene Katalysatoren eingesetzt werden. Beispielsweise kann der Katalysator mindestens ein Element der 8.-10. Gruppe des Periodensystems der Elemente enthalten. Optional können auch weitere Übergangsmetalle als Katalysatoren für diese Hydrierung eingesetzt werden, insbesondere Kupfer, und/oder Chrom und/oder mit mindestens einem weiteren Metall der 8. -10. Gruppe des Periodensystems der Elemente.

Bei heterogenen Katalysatoren können die oben genannten Metalle mit anderen Metallen oder Moderatoren modifiziert sein. So werden zum Beispiel heterogene Palladiumkatalysatoren oftmals durch Zusatz von Schwefel oder Kohlenmonoxid in ihrer Aktivität und Selektivität modifiziert. Kupfer-Katalysatoren wird oftmals ein Anteil an Chrom zugesetzt.

Der Einsatz von geträgerten Katalysatoren ist in der Regel vorteilhaft, da geringere Metallmengen benötigt werden und über die Beschaffenheit des Trägers zusätzlich die Eigenschaften des Katalysators beeinflusst werden können. Als Trägermaterialien haben sich beispielsweise Aktivkohle, Aluminiumoxid, Siliziumdioxid, Silizium-Aluminium-Oxid, Bariumcarbonat, Bariumsulfat und Kieselgur bewährt.

Die Hydrierungen werden bei Temperaturen von 0 bis 400 °C, bevorzugt von 20 bis 200 °C durchgeführt Der Druck beträgt dabei von 0,01 bis 300 bar, bevorzugt von 0,1 bis 125 bar, besonders bevorzugt von 1 bis 64 bar.

Die Hydrierung des 1-Methoxy-2,7-octadiens zum 1-Methoxyoctan in der Flüssigphase, egal ob homogen oder heterogen katalysiert, kann ohne oder in Gegenwart weiterer Komponenten erfolgen. Als weitere Komponenten kommen dabei noch nicht abgetrennte Edukte und Nebenprodukte aus Stufe a) und gegebenenfalls zugesetzte Lösemittel in Betracht. Noch vorhandene Edukte der Stufe a) können beispielsweise Methanol oder C4-Kohlenwasserstoffe sein, typische Nebenprodukte der Telomerisationsreaktion sind 3-Methoxy-1,7-octadien, 1,3,7-Octatriene, 1,7-Octadien, 1,6-Octadiene und Vinylcyclohexen.

Komponenten aus Stufe a), die in der Hydrierung anwesend sind, werden gegebenenfalls selbst ganz oder teilweise hydriert. So wird bei kompletter Hydrierung beispielsweise aus 3-Methoxy-1,7-octadien 3-Methoxyoctan, aus 1,3,7-Octatrien, 1,7-Octadien, 1,6-Octadien jeweils Octan, aus Vinylcyclohexen Ethylcyclohexan, aus 1,3-Butadien und n-Butenen Butan erhalten. Beispiele für Lösungsmittel, die in der Hydrierung zusätzlich zugesetzt werden können, sind aliphatische, cycloaliphatische und aromatische Kohlenwasserstoffe (Octan, Ethylcyclohexan), Alkohole (Methanol) und Ether (Dimethylether, Methyloctylether, 3-Methoxyoctan). Die Lösemittel kommen allein oder als Mischungen verschiedener Lösemittel zum Einsatz. Bevorzugt wird die Hydrierung ohne Zugabe von zusätzlichen Lösungsmitteln durchgeführt

Bei den Hydrierungen in der Gasphase können neben Wasserstoff und Substrat noch andere Gase anwesend sein. Beispielsweise können Stickstoff und/oder Argon, aber auch unter den Hydrierbedingungen gasförmige Alkane wie beispielsweise Methan, Propan oder Butan zugesetzt werden oder bereits im Hydriergas vorhanden sein.

Die Hydrierung in Schritt b) des erfindungsgemäßen Verfahrens kann kontinuierlich, semikontinuierlich oder diskontinuierlich (batchweise) erfolgen. Bevorzugt wird die kontinuierliche Verfahrensführung.

Vorzugsweise wird in Schritt b) des erfindungsgemäßen Verfahrens ein möglichst vollständiger Umsatz des 1-Methoxy-2,7-Octadiens angestrebt Der Umsatz liegt bevorzugt bei größer 98 %, insbesondere größer 99,5 %.

In einer bevorzugten Ausführungsform des erfindungsgemäßen Verfahrens wird die Hydrierung in der Flüssigphase an einem heterogenen Palladiumträgerkatalysator, der vorzugsweise von 0,01 bis 5 Gewichtsprozenten (Gew.-%) Palladium aufweist, durchgeführt. Der Druck beträgt dabei bevorzugt von 1 bis 64 bar und die Temperatur von 10 bis 140 °C. Die Hydrierung erfolgt in zwei Stufen, wobei die beiden Stufen optional mit einer Produktrückführung betrieben werden können.

Als Rohstoff für die Stufe c) des erfindungsgemäßen Verfahrens wird bevorzugt 1-Methoxyoctan hoher Reinheit eingesetzt Bevorzugt liegt der Gehalt an 1-Methoxyoctan bei > 99 Gew.-%. Zur Erreichung dieses Reinheitsgrades ist es zweckmäßig, andere Komponenten abzutrennen. Dies kann beispielsweise destillativ nach der Hydrierung, vor der Hydrierung oder sowohl vor als auch nach der Hydrierung im Prozess erfolgen. Im Austrag aus der Telomerisation, Stufe a) enthaltene C₄-Kohlenwasserstoffe werden bevorzugt vor der Hydrierung abgetrennt. Andere Komponenten wie Methanol, C₈-Kohlenwasserstoffe oder 3-Methoxy-1,7-octadien können vor oder nach (in der Regel dann in gesättigter Form) der Hydrierung entfernt werden.

In einer bevorzugten Ausführungsform des erfindungsgemäßen Verfahrens umfasst der Schritt a) eine Verfahrensstufe k) bei der nach der katalytischen Umsetzung C₄-Kohlenwasserstoffe destillativ abgetrennt werden. Der restliche Strom, der einen Gehalt an C4-Kohlenwasserstoffen von kleiner 5-Gew.% ausweist, wird in die Stufe b) geführt. Bei dieser Abtrennung wird auch ein Teil des im Strom enthaltenen Methanols als Azeotrop mit den C₄-Kohlenwasserstoffen entfernt (ca. 3 bis 6 Gew.-% Methanol-Anteil im C₄-Strom). Die zurückbleibende Mischung enthält hauptsächlich 1-Methoxy-2,7-octadien und Methanol, in Summe mit einem Anteil von > 80 Gew.-%. Nebenkomponenten sind, neben ggf. vorhandenen Restmengen an C₄-Kohlenwasserstoffen, hauptsächlich 3-Methoxy-1,7-octadien, 1,3,7-Octatrien, 1,7-Octadien, 1,6-Octadien und Vinylcyclohexen. Diese Mischung wird einer Hydrierung gemäß Schritt b) zugeführt, in der, neben der Hydrierung von 1-Methoxy-2,7-octadien zu 1-Methoxyoctan, auch die Nebenkomponenten zu 3-Methoxyoctan, n-Octan, Ethylcyclohexan und ggf. Ethylcyclohexen umgesetzt werden.

Der Austrag (Strom) der Hydrierung gemäß Schritt b) kann in einer bevorzugten Ausführungsart in einer Verfahrenstufe 1) anschließend destillativ aufgereinigt werden, wobei eine Leichtsiederfraktion, enthaltend Methanol, 3-Methoxyoctan und C₈-Kohlenwasserstoffe, insbesondere n-Octan, Ethylcyclohexan und Ethylcyclohexen, abgetrennt wird. Waren im Zulauf zur Hydrierung C₄-Kohlenwasserstoffe enthalten, werden auch diese hydriert und fallen zusammen mit der Leichtsiederfraktion, ggf. als Abgasstrom, am Kopf der Destillationskolonne an. Neben der Leichtsiederfraktion wird eine, 1-Methoxyoctan enthaltende, Schwersiederfraktion erhalten, die in Stufe c) geführt wird.

Der Vorteil bei dieser Aufarbeitung liegt unter anderem darin, dass sich durch die Hydrierung die Anzahl der als Nebenprodukte auftretenden Komponenten verringert, was eine weitere Aufarbeitung dieses Stoffstroms vereinfacht.

Die bei der Abtrennung der C4-Kohlenwasserstoffe gemäß Verfahrensstufe k) erhaltene Fraktion von C₄-Kohlenwasserstoffen, die einen Anteil an Methanol von ca. 3 bis 6 % aufweisen kann, kann bevorzugt einer Selektiv-Hydrierung, Verfahrensstufe m), zugeführt werden, in der Reste an 1,3-Butadien zu 1-Buten und 2-Butenen umgesetzt werden. Derartige Hydrierungen sind Stand der Technik. Bevorzugt erfolgt die Hydrierung in der Flüssigphase an heterogenen Palladium-Trägerkatalysatoren.

Der Austrag dieser Hydrierung (Verfahrensstufe m) kann dann beispielsweise einer Verätherung zugeführt werden, in der das Methanol mit dem in den C4-Strom enthaltenen Isobuten zu Methyl-tert.-butylether umgesetzt wird. Auch diese Umsetzung erfolgt nach in der Technik bekannten Verfahren, meist unter Katalyse von Ionentauschern. Für einen vollständigen Umsatz des Isobutens muss ggf. zusätzlich Methanol zugefügt werden.

Als eine Alternative kann der Austrag der Hydrierung (Verfahrensstufe m) zur Entfernung des Methanols in einer Verfahrensstufe n) mit Wasser gewaschen werden. Dabei erhält man eine im wesentlichen methanolfreie organische Phase, die handelsüblichem Raffinat I entspricht, und eine wässrige Phase. Die wässrige, Methanol-haltige Phase wird bevorzugt destillativ in Methanol und Wasser aufgearbeitet, das Wasser in die Extraktion (Verfahrensstufe n), das Methanol ganz oder teilweise in Stufe a) des erfindungsgemäßen Verfahrens zurückgeführt. Zur Herstellung dieses Raffinats I kann auch erst die Extraktion (Verfahrensstufe n) mit Wasser und anschließend eine selektive Hydrierung (Verfahrensstufe m) der Diene des C4-Stroms erfolgen.

Das Raffinat I kann entsprechend den bekannten Verfahren weiter verarbeitet werden, beispielsweise zur Herstellung von tert.-Butylalkohol, Diisobuten (oder Isooctan), Methyl-tert.-butylether, 1-Buten oder C4-Di- und Oligomeren, wie sie z.B. in DE 101 02 082, DE 25 38 036, DE 39 14 817, DE 103 02 457 oder DE 103 06 214 beschrieben werden.

In Verfahrensschritt c) wird das so erhaltene 1-Methoxyoctan an einem Katalysator zu Methanol und 1-Octen gespalten. Als Nebenprodukte können dabei auch Dimethylether (DME) und Wasser entstehen.

Die Spaltungsreaktion wird in Anwesenheit von heterogenen Katalysatoren durchgeführt. Bevorzugt werden Katalysatoren wie Aluminiumoxid, Kieselsäure, Silikate, basische Katalysatoren, Aluminium-haltige Kieselsäuren, Tonerde oder Zeolithe eingesetzt. Als basische Katalysatoren kommen bevorzugt Kontakte zum Einsatz, die in der deutschen Patentanmeldung mit der Amneldenummer DE 102 57 499 beschrieben sind. Die Spaltungsreaktion wird bei einer Temperatur zwischen 100 und 800 °C, bevorzugt zwischen 150 und 600 °C, besonders bevorzugt zwischen 250 und 500 °C durchgeführt. Der hierbei eingesetzte Druck beträgt 0,05 bis 300 bar, bevorzugt 1 bis 25 bar, besonders bevorzugt 1 bis 5 bar.
Das gemäß den Stufen a) bis c) erhaltene Spaltprodukt kann in verschiedenen Verfahrensvarianten weiter verarbeitet werden. Im einfachsten Fall kann das Spaltprodukt aus c) direkt destillativ in eine dampfförmige Leichtsiederfraktion, enthaltend mindestens 1-Octen und Wasser und eine flüssige Schwersiederfraktion, enthaltend mindestens 1-Octen und 1-Methoxyoctan getrennt werden (Verfahrensstufe d)).

Da häufig im Spaltprodukt DME und Wasser enthalten sind, wird jedoch bevorzugt zunächst ein Teil, vorzugsweise der größte Teil des DME destillativ vom Spaltprodukt abgetrennt Dies kann in einer Stufe d1) erfolgen, wobei das Spaltprodukt aus c) destillativ in eine Leichtsiederfraktion, enthaltend zumindest DME und eine Schwersiederfraktion getrennt und die Schwersiederfraktion in die Stufe d) geführt wird. Weist die Schwersiederfraktion aus d1) Methanol auf, so kann es vorteilhaft sein, diese mit Wasser zu waschen, wobei nach Phasentrennung (z.B. in einem Phasenabscheider) ein Methanolhaltiger wässriger Strom und ein unpolarer Strom erhalten werden, und der unpolare Strom in die Stufe d) geführt wird.

In einer anderen Ausführungsart des Erfindungsgemäßen Verfahrens wird das Spaltprodukt aus Stufe c), welches Methanol aufweist, zunächst in einem Verfahrensschritt d2) mit Wasser, z. B. mittels eines Dekanters oder einer Gegenstromextraktion unter Erhalt eines Methanol-haltigen wässrigen und eines unpolaren Stroms gewaschen. Der unpolare Strom kann dann ganz oder teilweise in die Stufe d) geführt werden. Mit diesem Verfahrensschritt wird das bei der Spaltung entstandene Methanol weitgehend abgetrennt. Die Extraktion wird vorzugsweise bei einer Temperatur von 10 bis 75 °C und einem Massenverhältnis zwischen dem zu reinigenden Strom und Wasser von 1 : 10 bis 10 : 1 durchgeführt.

Weist der unpolare Strom aus Verfahrensschritt d2) auch noch DME auf, so kann dieser destillativ in eine Leichtsiederfraktion, enthaltend zumindest DME und eine Schwersiederfraktion getrennt werden, wobei die Schwersiederfraktion in die Stufe d) geführt wird. Die destillative Abtrennung kann z.B. durch Überführung des unpolaren Stroms in Stufe d1) erfolgen. Wird keine Stufe d1) durchlaufen, kann am Kopf der Kolonne der Stufe d) DME, bevorzugt als gasförmiger Abgasstrom (nur teilweise Kondensation der dampfförmigen Leichtsiederfraktion) abgenommen werden.

Die Stufen d), d1) und d2) können so verschaltet sein, dass die Ströme oder Teilströme davon alle oder einen Teil der Stufen gegebenenfalls mehrfach durchlaufen. Auf diese Weise kann eine besonders gute Abtrennung von DME und Methanol erreicht werden.

Der in den Stufen d), d1) oder d2) abgetrennte Dimethylether (DME) kann beispielsweise als Heizgas (thermische Verwertung), als Rohstoff für chemische Prozesse (beispielsweise Olefinsynthesen) oder Brennstoffzellen oder als Treibgas eingesetzt werden. Je nach Verwendungszweck sind die Anforderung an die Reinheit des DME unterschiedlich. In einer bevorzugten Ausführungsform der Stufe d1) wird der DME in einer Reinheit > 99 %, besonders > 99,9 %, ganz besonders > 99,99 % gewonnen und als Treibgas eingesetzt.

Die Trennstufen d), e), f) werden bevorzugt in einer Destillationskolonne, die bei einem Druck von 0,5 bis 10, bevorzugt bei einem Druck von 2 bis 4 bar betrieben wird, in einem Kopfkondensator mit einer Betriebstemperatur von ca. 15 bis 75 °C und in einem Phasentrennbehälter (Dekanter) durchgeführt

In Stufe e) werden die in Stufe d) erhaltenen Leichtsieder ganz oder teilweise im Kopfkondensator kondensiert, die flüssige Phase in den Dekanter überführt und im Dekanter in eine polare und unpolare Phase getrennt. Optional fällt bei der Kondensation eine Gasphase (z. B. DME) an, die abgezogen wird (im Fall der Teilkondensation). Ist das Kondensat nicht zweiphasig, ist die Zugabe einer entsprechenden Menge Wasser in der Destillationsstufe d) oder im Dekanter zweckmäßig.

Die organische (unpolare) Phase des Dekanters wird vollständig in die Kolonne rückgeführt, während die wässrige Phase anderweitig weiter verwendet wird.

Durch dieses Verfahren werden Verluste an 1-Octen, die sich sonst aus der Bildung des Minimum-Azeotrops aus Wasser und 1-Octen ergeben würden, vermieden.

Als Schwersieder der Destillation in Stufe d) wird ein Gemisch, enthaltend die Octenisomere, 1-Methoxyoctan und in geringeren Mengen Nebenkomponenten wie 1-Octanol, C9+ Kohlenwasserstoffe (Kohlenwasserstoffe mit größer gleich 9 Kohlenstoffatomen), erhalten.

Dieses Gemisch wird gemäß Verfahrensstufe g) des erfindungsgemäßen Verfahrens in eine 1-Octen-haltige und eine 1-Methoxyoctan-haltige Fraktion aufgetrennt. Diese Destillation wird bei 50 bis 250°C und einem Druck von 0,1 bis 5 bar durchgeführt.

Der so erhaltene 1-Octen-Strom kann noch weitere Octen- und Nonenisomere aufweisen und ist für viele Anwendungen bereits ausreichend. Die 1-Octen-Konzentration in diesem Strom beträgt 80 bis 98 Gew.-%.

Soll 1-Octen mit einer Reinheit von über 90 Gew.% hergestellt werden, so wird zweckmäßiger Weise in einer Verfahrensstufe h) die 1-0cten-haltige Fraktion aus g) in eine Fraktion, enthaltend mindestens 1-Octen und eine Fraktion, enthaltend mindestens C₈- und/oder C₉-Olefine getrennt Die Abtrennung der unerwünschten Octen-Isomeren bzw. der Nonene erfolgt bevorzugt bei einer Temperatur zwischen 50 und 250 °C und einem Druck von 0,1 bis 5 bar. Das Zielprodukt 1-Octen wird hier als Kopfprodukt in einer Reinheit von> 90 Massen-%, bevorzugt > 95 Gew.-%, besonders bevorzugt 98,5 Gew.-% gewonnen. Die als Sumpfprodukt abgetrennten C₈-, C₉-Kohlenwasserstoffe können z. B. als Rohstoffe bei der Herstellung von Weichmacheralkohelen eingesetzt werden.

Das im Spaltprozess c) nicht umgesetzte 1-Methoxyoctan fällt mit weiteren Schwersiedern in der Destillation gemäß Verfahrensstufe g) als Sumpfprodukt an. Dieser Stoffstrom wird bevorzugt in die katalytische Spaltung c) zurückgeführt, wobei sich die genannten Schwersieder beispielsweise Dioctylether und andere Kohlenwasserstoffe am einfachsten durch einen geringen Teilstrom ausschleusen lassen. Optional ist es möglich, die 1-Methoxyoctan-haltige Fraktion aus g) in eine Leichtsiederfraktion enthaltend mindestens 1-Methoxyoctan und eine Schwersiederfraktion, enthaltend mindestens Dioctylether aufzutrennen (Verfahrensstufe i). Diese Destillation wird bevorzugt bei einer Temperatur von 100 bis 300 °C bei einem Druck von 0,1 bis 2,5 bar durchgeführt. Das so erhaltene 1-Methoxyoctan weist eine Reinheit von 90 bis 100 Gew.% auf und wird zweckmäßiger Weise in die Spaltungsreaktion c) zurückgeführt. Die Dioctylether aufweisende Schwersiederfraktion aus Stufe i) kann einer thermischen Verwertung oder einer anderen Verwertung, beispielsweise Herstellung von Synthesegas, zugeführt werden.

Werden bei dem erfindungsgemäßen Verfahren Methanol-haltige, wässrige Ströme erhalten, wie sie z.B. in den Verfahrensschritten d1), d2), e) und n), z.B. durch Extraktion erhalten werden können, so kann es vorteilhaft sein, wenn diese in einer Verfahrensstufe o) aufgearbeitet werden, so dass Methanol und/oder Wasser abgetrennt werden. Das Methanol kann ganz oder teilweise in die Telomerisation des Schrittes a) rückgeführt werden. Bevorzugtes Verfahren zur Abtrennung ist die destillative Trennung. Sollte neben der Wasser/Methanol-Phase noch eine zweite, organische Phase vorliegen, wird diese vor der Destillation bevorzugt abgetrennt und die wässrige Phase destillativ in eine Methanol aufweisende Leichtsiederfraktion und eine Wasser aufweisende Schwersiederfraktion getrennt

Die Aufarbeitung der Methanol-haltigen, wässrigen Ströme, wie sie z.B. aus der Stufe d2) erhalten werden, kann auch zusammen mit weiteren Strömen des Prozesses erfolgen. Als weitere Ströme sind dabei insbesondere die wässrige Phase aus Stufe e), die Methanol enthaltende Leichtsiederfraktion aus Verfahrensstufe 1) und die wässrige, Methanol-haltige Phase aus Verfahrensschritt n), geeignet

Besonders vorteilhaft ist es, die Ströme so aufzuarbeiten, dass man nur eine Methanol-Wasser Mischung erhält, die in einer zentralen Einheit wieder in Methanol und Wasser aufgearbeitet wird. Das Wasser kann dann in die im erfindungsgemäßen Verfahren vorhandenen Extraktionen und Methanol in Schritt a) des erfindungsgemäßen Verfahrens zurückgeführt werden. Beispiele für die gemeinsame Aufarbeitung von Methanol-haltigen Strömen werden bei den nachfolgend aufgeführten Verfahrensvarianten diskutiert

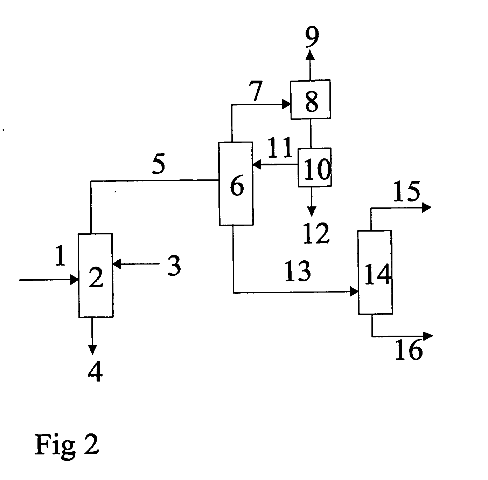

Eine Verfahrensvariante soll im Folgenden anhand der Figur 2 erläutert werden: Strom (1) bezeichnet das Spaltprodukt, das aus Verfahrensstufe c) erhalten wird und typischer Weise die in der Tabelle 1 angegebene Zusammensetzung aufweist. In der Waschstufe (2) (Verfahrensstufe d2)) wird das Spaltprodukt mit Wasser (3) unter Erhalt einer wässrigen Lösung (4) ausgewaschen. Der unpolare Stoffstrom (5) wird anschließend in der Destillationskolonne (6) (Verfahrensstufe d)) in eine Leichtsiederfraktion (7), die weitgehend aus DME, Wasser und Octenen besteht und der Schwersiederfraktion (13), die den größten Teil des 1-Octen, Schwersieder und 1-Methoxyoctan enthält, getrennt. Die Leichtsiederfraktion (7) wird nach partieller Kondensation (8) unter Ausschleusung eines gasförmigen Stroms (9) (DMB) im Dekanter (10) in eine leichte, organische Phase (11), die in die Destillationskolonne (6) rückgeführt wird (Verfahrensstufe f)), und eine schwere, wässrige Phase (12), die ausgeschleust wird, getrennt (Verfahrensstufe e)). Gegebenenfalls kann der Strom (12) ganz oder teilweise dem Strom (3) oder dem Strom (4) zugesetzt werden. Strom (13) wird in einer weiteren Destillationskolonne (14) auf das Zielprodukt, 1-Octen (15) und einer 1-Methoxyoctan-haltige Fraktion (16) aufgetrennt (Verfahrensstufe g)).

Es ist möglich, auf die Wäsche (2) unter Zugabe von Wasser (3) zu verzichten, und statt dessen einen Vordekanter zur Abtrennung einer wässrigen Phase einzusetzen.

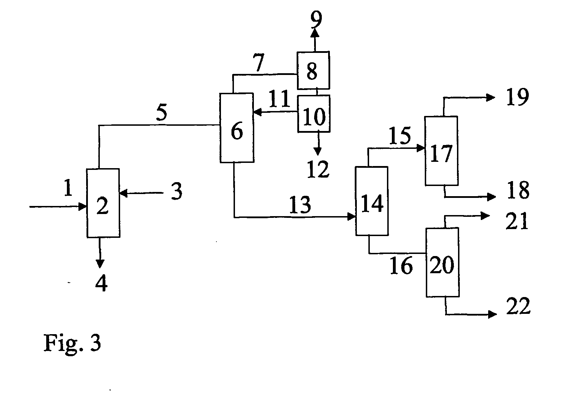

Fig. 3 entspricht im Wesentlichen Fig. 2 ist aber um die weitere Aufreinigung des 1-Octens bzw. des optional rückzufübrenden 1-Methoxyoctan ergänzt worden. Die 1-Octen-haltige Fraktion (15) wird in einer Destillationskolonne (17), in hochreines 1-Octen (19) und Schwersiederfraktion (18) getrennt (Verfahrensstufe h)), wobei Strom (18) die unerwünschten Octenisomere wie 2-Octene, 3-Octene und 4-Octene sowie die als Nebenprodukt anfallenden Nonene enthält. Die 1-Methoxyoctan-haltige Fraktion (16) wird zur Vermeidung der Anreicherung von schwersiedenden Nebenprodukten in einer Destillationskolonne in 1-Methoxyoctan (21) und die Schwersiederfraktion (22) aufgetrennt (Verfahrensstufe i)). Strom (21) wird zweckmäßig in die Spaltungsreaktion gemäß Verfahrensschritt c) rückgeführt.

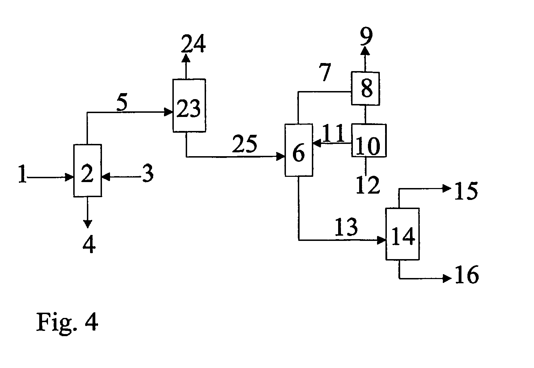

In Fig. 4 wird Strom (5) aus Verfahrensstufe d2) zunächst in Kolonne (23) von DME (24) befreit (Verfahrensstufe d1)); die Schwersieder (25) werden entweder wie in der Fig. 2 oder in Fig. 3 (als Strom 5 bezeichnet) weiterverarbeitet Fig. 4 beschreibt also eine Variante des erfindungsgemäßen Verfahrens, bei der die Verfahrensstufen in der Reihenfolge d2), d1) und d) durchgeführt werden.

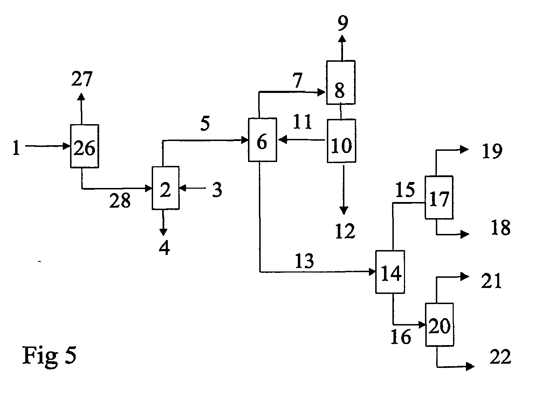

Fig. 5 zeigt eine weitere Variante, bei der DME (27) bereits vor der Wasserwäsche (2) in einer Kolonne (26) entfernt wird (Verfahrensstufe d1)). Der in Kolonne (26) anfallende Sumpfstrom (28) wird in die Wasserwäsche (2) (Verfahrensstufe d2)) geführt und anschließend wie in Fig. 3 beschrieben, aufgearbeitet Fig. 5 beschreibt also eine Verfahrensvariante, bei der die Verfahrensstufen in der Reihenfolge d1), d2) und d) durchgeführt werden.

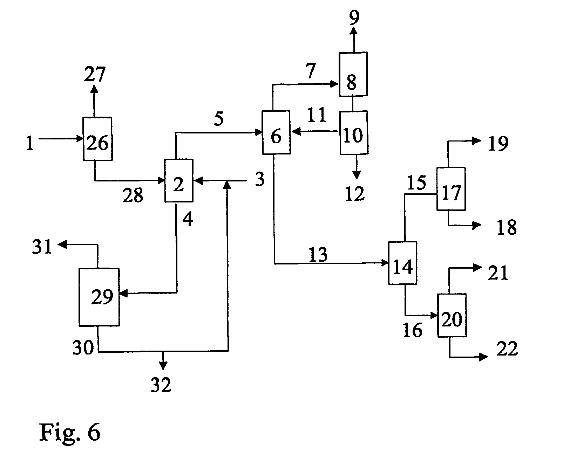

Fig. 6 zeigt eine um eine Wasser/Methanol-Aufarbeitung erweiterte Variante der Fig. 5 (eine mögliche Ausführungsform der Verfahrensstufe o)), in der die wässrige Lösung (4), die gegebenenfalls mit Strom (12) vereinigt wird, in einer weiteren Kolonne (29) in eine hauptsächlich Wasser enthaltende Sumpffraktion (30) und eine methanolhaltige Kopffraktion (31) getrennt wird. Der Strom (30) kann ganz oder teilweise als Wasser in die Waschstufe (2) zurückgeführt werden. Strom (32) kennzeichnet einen Ausschleusestrom.

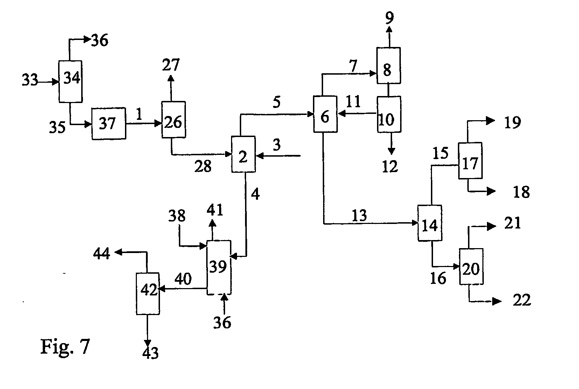

Fig. 7 zeigt eine weitere Variante der Fig. 5 mit einer weiteren möglichen Ausführungsform der Verfahrensstufe o), bei welcher ein Methanolhaltiger Strom aus der Verfahrensstufe 1) ebenfalls aufgearbeitet wird. Ein Austrag der Hydrierung (Strom 33), der neben dem 1-Methoxyoctan noch Methanol, 3-Methoxyoctan und C8-Kohlenwasserstoffe enthält, wird in einer Kolonne (34) in eine Sumpffraktion (35), die 1-Methoxyoctan enthält, und eine Kopffraktion, die hauptsächlich Methanol, 3-Methoxyoctan und C8-Kohlenwasserstoffe (36) enthält, getrennt (Verfahrensstufe 1)). Strom (35) wird der Spaltung (37) zugeführt, aus der das Spaltprodukt (1) erhalten wird. Aus diesem wird in Kolonne (26) DME (27) entfernt. Der in Kolonne (26) anfallende Sumpfstrom (28) wird in (2) in eine organische Phase (5) und eine wässrige Phase (4) getrennt. Falls notwendig, wird zusätzlich Wasser (3) zugegeben (technisch kann dies beispielsweise als Dekanter, Mixer-Settler oder Extraktionskolonne ausgeführt sein). Die organische Phase (5) wird wie in Fig. 3 beschrieben aufgearbeitet. Die wässrige Phase (4) wird zusammen mit Strom (36) und gegebenenfalls zusammen mit Strom (12) (nicht abgebildet) in eine Extraktion (39) geführt, der gegebenenfalls noch zusätzlich Wasser (38) zugeführt wird. In der Extraktion (39) wird eine wässrige Phase (40) und eine organische Phase (41) gewonnen. Die wässrig Phase (40), die auch den Großteil an Methanol enthält, wird der Kolonne (42) zugeführt, wo sie in eine hauptsächlich Wasser enthaltende Sumpf&aktion (43) und eine methanolhaltige Kopffraktion (44) getrennt wird. Der Strom (43) kann ganz oder teilweise als Wasser in Stufe (2) (als Strom (3)) oder in Stufe (39) (als Strom (38)) zurückgeführt werden. Der Strom (44), der hauptsächlich Methanol enthält, kann ganz oder teilweise in die Telomerisation zurückgeführt werden.

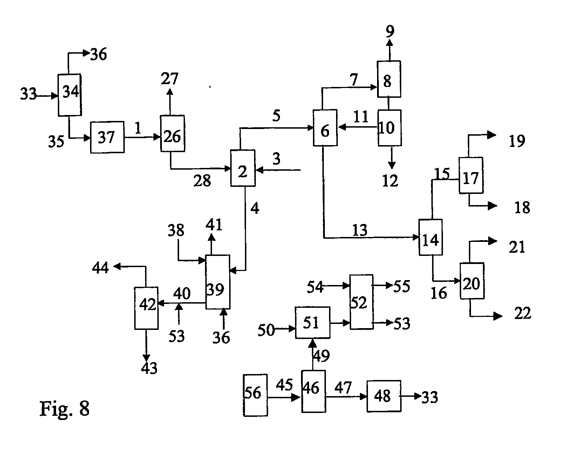

Fig. 8 zeigt eine erweiterte Variante von Fig. 7. Vom Austrag (45) der Telomerisation (56) des erfindungsgemäßen Verfahrens wird in einer Trenneinheit (46) (Verfahrensstufe k)) ein C4-Methanol-Gemisch (49) abgetrennt und in eine Selektivhydrierung (51) geführt. Der verbleibende Strom (47) wird der Hydrierung (48) (Verfahrensstufe b)) zugeführt, aus der der Strom (33) resultiert In der Selektivhydrierung (51) (Verfahrensstufe m)) werden Reste an 1,3-Butadien mit Wasserstoff (50) zu Butenen umgesetzt. Der Austrag der Selektivhydrierung wird einer Wasserwäsche (52) (Verfahrensstufe n)) zugeleitet Hier wird mit Wasser (54) das im C4 enthaltene Methanol entfernt Die dabei erhaltene Methanol/Wasser-Mischung (53) kann in Kolonne (42) zusammen mit anderen Prozessströmen aufgereinigt werden. Der Methanol-freie C4-Strom (55) entspricht in seiner Zusammensetzung handelsüblichem Raffinat I und steht für andere Anwendungen zu Verfügung. Das für die Extraktion eingesetzte Wasser (54) kann aus dem Strom (43) entnommen werden.

Der Übersichtlichkeit halber sind in der nachfolgenden Auflistung noch einmal die Hauptfunktionen der im Text unter den verschiedenen Ausführungsformen des erfindungsgemäßen Verfahrens erwähnten Verfahrensstufen aufgeführt.
a) Katalytisches Umsetzen eines Butadien-haltigen Stroms mit Methanol unter Erhalt eines mindestens 1-Methoxy-2,7-Octadien-haltigen Stroms.
b) Katalytische Hydrierung des 1-Methoxy-2,7-Octadien-haltigen Stroms, der in Stufe a) gewonnen wird, zu einem mindestens 1-Methoxyoctan-haltigen Strom.
c) Katalytische Spaltung zumindest eines Teils des 1-Methoxyoctans zu einem Spaltprodukt, das mindestens Wasser, 1-Octen und gegebenenfalls nicht umgesetztes 1-Methoxyoctan enthält.
d) Destillative Auftrennung des Spaltprodukts aus Stufe c) in eine dampfförmige Leichtsiederfiaktion, enthaltend mindestens 1-Octen und Wasser und eine flüssige Schwersiederfraktion, enthaltend mindestens 1-Octen und 1-Methoxyoctan.
   d1) Abtrennung von DME
   d2) Wäsche mit Wasser um Methanol zu entfernen
e) Ganz oder teilweise Kondensation der Leichtsiederfraktion aus Stufe d) und Trennung des Kondensats in eine wässrige und eine 1-Octen enthaltende, unpolare Phase.
f) Rückführung der 1-Octen-haltigen unpolaren Phase aus Stufe e) in die Stufe d).
g) Trennung der Schwersiederfraktion aus d) in eine 1-Octen-haltige und eine 1-Methoxyoctan-haltige Fraktion.
h) Trennung der 1-Octen-haltigen Fraktion aus g) in eine Fraktion, enthaltend mindestens 1-Octen und eine Fraktion, enthaltend mindestens C₈- und/oder C₉-Olefine.
i) Trennung der 1-Methoxy-octan-haltigen Fraktion aus Stufe g) in eine mindestens 1-Methoxy-octan-haltige Leichtsiederfraktion und eine mindestens Dioctylether enthaltende Schwersiederfraktion.
k) Teil der Verfahrensstufe a), bei der die nicht umgesetzten C4-Kohlenwasserstoffe abgetrennt werden. Aufgrund der Bildung von Azeotropen enthält dieser Strom noch Methanol.
l) Destillative Au&einigung des Austrags aus Stufe b), bei der Leichtsieder vom 1-Methoxyoctan abgetrennt werden.
m) Hydrierung von Resten an 1,3-Butadien zu Butenen.
n) Wäsche mit Wasser um Methanol aus C4-Kohlenwasserstoffen zu entfernen.
o) Rückgewinnung von Methanol aus wässrigen Methanolhaltigen Lösungen (verschiedene Ausführungsformen sind möglich).

Die im erfindungsgemäßen Verfahren eingesetzten Destillations- oder Extraktionskolonnen sind vorzugsweise Füllkörperkolonnen bzw. weisen Einbauten wie z. B. Glockenböden, Siebkolonnen oder Demesterpackungen auf.

Um eine ausreichende Trennschärfe zu erhalten, sollten die Destillationskolonnen von 75 bis 250, bevorzugt von 80 bis 100 theoretische Trennstufen für die Destillation gemäß Stufe h) aufweisen. Für die übrigen Kolonnen können von 5 bis 60 Stufen theoretische Trennstufen ausreichend sein. Die in Verfahrensschritt d) verwendete Kolonne weist bevorzugt ein Rücklaufverhältnis von 0,4 bis 0,9 auf. Entsprechend liegen die Rücklaufverhältnisse der Kolonne gemäß Stufe g) bevorzugt bei 0,6 bis 1,4; die zur 1-Octen-Aufreinigung eingesetzte Kolonne gemäß Stufe h) weist bevorzugt ein Rücklaufverhältnis von 4 bis 11 und die Kolonne gemäß Verfahrensstufe i) bevorzugt eines von 1,9 bis 3,7 auf.

Die nachfolgenden Beispiele sollen die vorliegende Erfindung beschreiben, ohne dass der Schutzumfang, der sich aus den Ansprüchen und der Beschreibung ergibt, auf diese Beispiele beschränkt sein soll.

### Beispiel 1:

Für das erfindungsgemäße Verfahren gemäß Fig. 5 wurde eine Planungsauslegung erstellt, in der Stoffströme und Apparateparameter dimensioniert wurden. Als Simulationssoftware wurde mit einem AspenPlus-Simulationsmodell, Version 11.1, Firma Aspentech gerechnet. Die Stoffdaten der in der Aspen-Datenbank nicht vorhandenen Komponenten wurden auf Basis der Molekülstruktur mit Standardmethoden (der Aspen-Simulationssoftware) parametrisiert. Für 1-Methoxyoctan wurde die Parametrisierung durch Anpassung an die Messdaten der Dampfdruckkurve verfeinert. Die Messdaten zur Ermittlung der Dampfdruckkurve wurde auf übliche Weise bestimmt.

**Tabelle 2: Messdaten Dampfdruckkurve**

| Dampfdruck 1-Methoxyoctan | |
|---|---|
| Temperatur | Druck |
| [°C] | [mbar] |
| 52,33 | 9,6 |
| 57,42 | 12,7 |
| 61,06 | 15,5 |
| 65,97 | 20,1 |
| 74,21 | 30.3 |
| 85,08 | 50,4 |
| 92,70 | 70,3 |
| 99,38 | 91,5 |
| 104,12 | 110,5 |
| 112,30 | 150,8 |
| 126,36 | 249,5 |
| 140,45 | 396,1 |
| 153,95 | 595,3 |
| 164,21 | 795,3 |
| 173,18 | 1013,9 |

Die Parameter der Destillationskolonnen sind in Tabelle 3 angegeben. Die Nummerierung der Kolonnen (Block) entspricht den Nummerierungen in Fig. 5.

**Tabelle 3: Kolonnenparameter**

| **Block** | **Stufenzahl** | **Kopfdruck** | **Rücklaufverhältnis** |
|---|---|---|---|
| | **-** | **bar** | **kg/kg** |
| **26** | 20 | 9.0 | 1.0 |
| **6** | 20 | 1.0 | 3.0 |
| **14** | 30 | 1.0 | 1.0 |
| **17** | 100 | 1.0 | 8.0 |
| **20** | 40 | 1.0 | 3.0 |

Die aus diesen Bedingungen resultierenden Stoffströme haben die in Tabelle 4 a und b aufgelisteten Zusammensetzungen. Die Stromnummern entsprechen wiederum den Bezeichnungen in Fig. 5.

**Tabelle 4a:**

| | | Strom-Nr. | | | | | | |
|---|---|---|---|---|---|---|---|---|
| | | **1** | **4** | **5** | **9** | **12** | **13** | **15** |
| Massenstrom | kg/h | 22500 | 1145 | 19287 | 7 | 320 | 18960 | 13028 |
| Konzentrationen | | | | | | | | |
| Dimethylether | kg/kg | 0.0928 | 0.0006 | 0.0010 | 0.7003 | 0.0462 | 0.0000 | 0.0000 |
| Methanol | kg/kg | 0.0278 | 0.3451 | 0.0119 | 0.1324 | 0.7161 | 0.0000 | 0.0000 |
| Wasser | kg/kg | 0.0363 | 0.6525 | 0.0036 | 0.0111 | 0.2171 | 0.0000 | 0.0000 |
| Org. Leichtsieder | kg/kg | 0.0004 | 0.0000 | 0.0005 | 0.0754 | 0.0056 | 0.0004 | 0.0005 |
| 1-Octen | kg/kg | 0.5393 | 0.0010 | 0.6291 | 0.0795 | 0.0147 | 0.6397 | 0.9309 |
| 3-/4-Octen | kg/kg | 0.0058 | 0.0000 | 0.0068 | 0.0005 | 0.0001 | 0.0069 | 0.0100 |
| 2-Octen | kg/kg | 0.0222 | 0.0000 | 0.0259 | 0.0008 | 0.0001 | 0.0263 | 0.0383 |
| Nonene | kg/kg | 0.0109 | 0.0000 | 0.0127 | 0.0000 | 0.0000 | 0.0129 | 0.0187 |
| Cyclooctan | kg/kg | 0.0012 | 0.0000 | 0.0014 | 0.0000 | 0.0000 | 0.0014 | 0.0012 |
| 1-Methoxyoctan | kg/kg | 0.2339 | 0.0002 | 0.2729 | 0.0000 | 0.0000 | 0.2776 | 0.0001 |
| 2-Octanol | kg/kg | 0.0010 | 0.0000 | 0.0012 | 0.0000 | 0.0000 | 0.0012 | 0.0002 |
| 1-Octanol | kg/kg | 0.0171 | 0.0005 | 0.0199 | 0.0000 | 0.0000 | 0.0203 | 0.0000 |
| C16-KWs | kg/kg | 0.0023 | 0.0000 | 0.0027 | 0.0000 | 0.0000 | 0.0027 | 0.0000 |
| Dioctylether | kg/kg | 0.0060 | 0.0000 | 0.0070 | 0.0000 | 0.0000 | 0.0071 | 0.0000 |
| Schwersieder | kg/kg | 0.0030 | 0.0000 | 0.0035 | 0.0000 | 0.0000 | 0.0036 | 0.0000 |

**Tabelle 4b:**

| | | Strom-Nr. | | | | | | |
|---|---|---|---|---|---|---|---|---|
| | | **16** | **18** | **19** | **21** | **22** | **27** | **28** |
| **Massenstrom** | kg/h | 5932 | 962 | 12066 | 5419 | 513 | 2068 | 20432 |
| **Konzentrationen** | | | | | | | | |
| Dimethylether | kg/kg | 0.0000 | 0.0000 | 0.0000 | 0.0000 | 0.0000 | 1.0000 | 0.0010 |
| Methanol | kg/kg | 0.0000 | 0.0000 | 0.0000 | 0.0000 | 0.0000 | 0.0000 | 0.0306 |
| Wasser | kg/kg | 0.0000 | 0.0000 | 0.0000 | 0.0000 | 0.0000 | 0.0000 | 0.0400 |
| Org. Leichtsieder | kg/kg | 0.0000 | 0.0000 | 0.0006 | 0.0000 | 0.0000 | 0.0000 | 0.0004 |
| 1-Octen | kg/kg | 0.0000 | 0.2521 | 0.9850 | 0.0000 | 0.0000 | 0.0000 | 0.5939 |
| 3-/4-Octen | kg/kg | 0.0000 | 0.0462 | 0.0071 | 0.0000 | 0.0000 | 0.0000 | 0.0064 |
| 2-Octen | kg/kg | 0.0000 | 0.4273 | 0.0073 | 0.0000 | 0.0000 | 0.0000 | 0.0244 |
| Nonene | kg/kg | 0.0002 | 0.2534 | 0.0000 | 0.0003 | 0.0000 | 0.0000 | 0.0120 |
| Cyclooctan | kg/kg | 0.0018 | 0.0168 | 0.0000 | 0.0020 | 0.0000 | 0.0000 | 0.0013 |
| 1-Methoxvoctan | kg/kg | 0.8870 | 0.0014 | 0.0000 | 0.9700 | 0.0103 | 0.0000 | 0.2576 |
| 2-Octanol | kg/kg | 0.0033 | 0.0029 | 0.0000 | 0.0036 | 0.0001 | 0.0000 | 0.0011 |
| 1-Octanol | kg/kg | 0.0647 | 0.0001 | 0.0000 | 0.0241 | 0.4941 | 0.0000 | 0.0188 |
| C16-KWs | kg/kg | 0.0087 | 0.0000 | 0.0000 | 0.0000 | 0.1009 | 0.0000 | 0.0025 |
| Dioctylether | kg/kg | 0.0228 | 0.0000 | 0.0000 | 0.0000 | 0.2631 | 0.0000 | 0.0066 |
| Schwersieder | kg/kg | 0.0114 | 0.0000 | 0.0000 | 0.0000 | 0.1306 | 0.0000 | 0.0033 |

### Beispiel 2 (Vergleichsbeispiel)

In Beispiel 2 wurde das Simulationsmodell verwendet um die gleiche Anlagenverschaltung wie in Beispiel 1, aber ohne Kopfdekanter (Block 10) der Trenneinheit 6, rechnerisch im Modell nachzustellen. Die Kolonnenparameter bleiben unverändert zu Beispiel 1. Die resultierenden Stoffströme haben die in den Tabellen 5a und 5b angegebenen Zusammensetzungen

**Tabelle 5a:**

| | | Strom-Nr. | | | | | | |
|---|---|---|---|---|---|---|---|---|
| | | **1** | **4** | **5** | **9** | **12** | **13** | **15** |
| Massenstrom | kg/h | 22500 | 1145 | 19287 | 8 | 362 | 18917 | 12985 |
| Konzentrationen | | | | | | | | |
| Dimethylether | kg/kg | 0.0928 | 0.0006 | 0.0010 | 0.5235 | 0.0429 | 0.0000 | 0.0000 |
| Methanol | kg/kg | 0.0278 | 0.3451 | 0.0119 | 0.3159 | 0.6251 | 0.0001 | 0.0001 |
| Wasser | kg/kg | 0.0363 | 0.6525 | 0.0036 | 0.0025 | 0.0065 | 0.0036 | 0.0052 |
| Org.Leichtsieder | kg/kg | 0.0004 | 0.0000 | 0.0005 | 0.0044 | 0.0020 | 0.0004 | 0.0006 |
| 1-Octen | kg/kg | 0.5393 | 0.0010 | 0.6291 | 0.1486 | 0.3122 | 0.6353 | 0.9256 |
| 3-/4-Octen | kg/kg | 0.0058 | 0.0000 | 0.0068 | 0.0014 | 0.0030 | 0.0068 | 0.0100 |
| 2-Octen | kg/kg | 0.0222 | 0.0000 | 0.0259 | 0.0037 | 0.0084 | 0.0262 | 0.0382 |
| Nonene | kg/kg | 0.0109 | 0.0000 | 0.0127 | 0.0000 | 0.0000 | 0.0130 | 0.0188 |
| Cyclooctan | kg/kg | 0.0012 | 0.0000 | 0.0014 | 0.0000 | 0.0000 | 0.0014 | 0.0012 |
| 1-Methoxvoctan | kg/kg | 0.2339 | 0.0002 | 0.2729 | 0.0000 | 0.0000 | 0.2782 | 0.0001 |
| 2-Octanol | kg/kg | 0.0010 | 0.0000 | 0.0012 | 0.0000 | 0.0000 | 0.0012 | 0.0002 |
| 1-Oclanol | kg/kg | 0.0171 | 0.0005 | 0.0199 | 0.0000 | 0.0000 | 0.0203 | 0.0000 |
| C16-KWs | kg/kg | 0.0023 | 0.0000 | 0.0027 | 0.0000 | 0.0000 | 0.0027 | 0.0000 |
| Dioctylether | kg/kg | 0.0060 | 0.0000 | 0.0070 | 0.0000 | 0.0000 | 0.0071 | 0.0000 |
| Schwersieder | kg/kg | 0.0030 | 0.0000 | 0.0035 | 0.0000 | 0.0000 | 0.0036 | 0.0000 |

**Tabelle 5b:**

| | | Strom-Nr. | | | | | | |
|---|---|---|---|---|---|---|---|---|
| | | **16** | **18** | **19** | **21** | **22** | **27** | **28** |
| Massenstrom | kg/h | 5932 | 1028 | 11958 | 5419 | 513 | 2068 | 20432 |
| Konzentrationen | | | | | | | | |
| Dimethylether | kg/kg | 0.0000 | 0.0000 | 0.0000 | 0.0000 | 0.0000 | 1.0000 | 0.0010 |
| Methanol | kg/kg | 0.0000 | 0.0000 | 0.0001 | 0.0000 | 0.0000 | 0.0000 | 0.0306 |
| Wasser | kg/kg | 0.0000 | 0.0000 | 0.0056 | 0.0000 | 0.0000 | 0.0000 | 0.0400 |
| Org. Leichtsieder | kg/kg | 0.0000 | 0.0000 | 0.0007 | 0.0000 | 0.0000 | 0.0000 | 0.0004 |
| 1-Octen | kg/kg | 0.0000 | 0.2339 | 0.9850 | 0.0000 | 0.0000 | 0.0000 | 0.5939 |
| 3-/4-Octen | kg/kg | 0.0000 | 0.0571 | 0.0059 | 0.0000 | 0.0000 | 0.0000 | 0.0064 |
| 2-Oclen | kg/kg | 0.0000 | 0.4523 | 0.0027 | 0.0000 | 0.9000 | 0.0000 | 0.0244 |
| Nonene | kg/kg | 0.0002 | 0.2372 | 0.0000 | 0.0003 | 0.0000 | 0.0000 | 0.0120 |
| Cyclooctan | kg/kg | 0.0019 | 0.0155 | 0.0000 | 0.0020 | 0.0000 | 0.0000 | 0.0013 |
| 1-Methoxyoctan | kg/kg | 0.8869 | 0.0013 | 0.0000 | 0.9700 | 0.0102 | 0.0000 | 0.2576 |
| 2-Octanol | kg/kg | 0.0033 | 0.0026 | 0.0000 | 0.0037 | 0.0001 | 0.0000 | 0.0011 |
| 1-Octanol | kg/kg | 0.0647 | 0.0001 | 0.0000 | 0.0240 | 0.4944 | 0.0000 | 0.0188 |
| C16-KWs | kg/kg | 0.0087 | 0.0000 | 0.0000 | 0.0000 | 0.1008 | 0.0000 | 0.0025 |
| Dioctylether | kg/kg | 0.0228 | 0.0000 | 0.0000 | 0.0000 | 0.2630 | 0.0000 | 0.0066 |
| Schwersieder | kg/kg | 0.0114 | 0.0000 | 0.0000 | 0.0000 | 0.1315 | 0.0000 | 0.0033 |

Es ist zu erkennen, dass der fehlende Kopfdekanter zu Verlusten des Produktes 1-Octen mit dem Strom 12 führt Hier gehen etwa 1 % 1-Octen verloren. Außerdem bleibt ein formaler Restgehalt von ca. 5600 ppm Wasser im Endprodukt 1-Octen, der in zusätzlichen Stufen entfernt werden müsste. Gemäß der Verschaltung des erfindungsgemäßen Verfahrens können hingegen sowohl Methanol als auch Wasser spezifikationsgerecht aus dem 1-Octen-Produkt abgetrennt werden.

### Beispiel 3:

Für das erfindungsgemäße Verfahren gemäß Fig. 7 wurde eine Planungsauslegung erstellt, in der Stoffströme und Apparateparameter dimensioniert wurden. Die Simulationssoftware und die Stoffdaten entsprechen denen aus Beispiel 1.

Die Parameter der Destillationskolonnen sind in Tabelle 6 angegeben. Die Nummerierung der Kolonnen (Block) entspricht den Nummerierungen in Fig. 7. Bei Block (39) handelt es sich um eine Extraktionskolonne.

**Tabelle 6: Kolonnenparameter**

| **Block** | **Stufenzahl** | **Kopfdruck** | **Rücklaufverhältnis** |
|---|---|---|---|
| | **-** | **bar** | **kg/kg** |
| **34** | 50 | 1.0 | 2.0 |
| **26** | 20 | 9.0 | 1.0 |
| **6** | 20 | 1.0 | 3.0 |
| **14** | 30 | 1.0 | 1.0 |
| **17** | 100 | 1.0 | 8.0 |
| **20** | 40 | 1.0 | 3.0 |
| **39** | 5 | 1.0 | - |
| **42** | 30 | 1.0 | 2.0 |

Die aus diesen Bedingungen resultierenden Stoffströme haben die in Tabelle 7 a, b und c aufgelisteten Zusammensetzungen. Die Stromnummern entsprechen wiederum den Bezeichnungen in Fig. 7.

**Tabelle 7a:**

| | Strom-Nr. | | | | | | | |
|---|---|---|---|---|---|---|---|---|
| | **1** | **4** | **5** | **9** | **12** | **13** | **15** | **16** |
| Massenstrom in kg/h | 22500 | 1145 | 19287 | 7 | 320 | 18960 | 13028 | 5932 |
| Konzentrationen in kg/kg | | | | | | | | |
| C4-KWs | 0.0000 | 0.0000 | 0.0000 | 0.0000 | 0.0000 | 0.0000 | 0.0000 | 0.0000 |
| Dimethylether | 0.0928 | 0.0006 | 0.0010 | 0.7003 | 0.0462 | 0.0000 | 0.0000 | 0.0000 |
| Methanol | 0.0278 | 0.3451 | 0.0119 | 0.1324 | 0.7161 | 0.0000 | 0.0000 | 0.0000 |
| Wasser | 0.0363 | 0.6525 | 0.0036 | 0.0111 | 0.2171 | 0.0000 | 0.0000 | 0.0000 |
| Org.Leichtsieder | 0.0004 | 0.0000 | 0.0005 | 0.0754 | 0.0056 | 0.0004 | 0.0005 | 0.0000 |
| 1-Octen | 0.5393 | 0.0010 | 0.6291 | 0.0795 | 0.0147 | 0.6397 | 0.9309 | 0.0000 |
| 3-/4-Octen | 0.0058 | 0.0000 | 0.0068 | 0.0005 | 0.0001 | 0.0069 | 0.0100 | 0.0000 |
| 2-Octen | 0.0222 | 0.0000 | 0.0259 | 0.0008 | 0.0001 | 0.0263 | 0.0383 | 0.0000 |
| n-Octan | 0.0001 | 0.0000 | 0.0001 | 0.0000 | 0.0000 | 0.0001 | 0.0002 | 0.0000 |
| Nonene | 0.0108 | 0.0000 | 0.0126 | 0.0000 | 0.0000 | 0.0128 | 0.0185 | 0.0002 |
| Cyclooctan | 0.0012 | 0.0000 | 0.0014 | 0.0000 | 0.0000 | 0.0014 | 0.0012 | 0.0018 |
| 3-Methoxyoctan | 0.0000 | 0.0000 | 0.0000 | 0.0000 | 0.0000 | 0.0000 | 0.0000 | 0.0000 |
| 1-Methoxvoctan | 0.2339 | 0.0002 | 0.2729 | 0.0000 | 0.0000 | 0.2776 | 0.0001 | 0.8870 |
| 2-Octanol | 0.0010 | 0.0000 | 0.0012 | 0.0000 | 0.0000 | 0.0012 | 0.0002 | 0.0033 |
| 1-Octanol | 0.0171 | 0.0005 | 0.0199 | 0.0000 | 0.0000 | 0.0203 | 0.0000 | 0.0647 |
| C16-KWs | 0.0023 | 0.0000 | 0.0027 | 0.0000 | 0.0000 | 0.0027 | 0.0000 | 0.0087 |
| Dioctylether | 0.0060 | 0.0000 | 0.0070 | 0.0000 | 0.0000 | 0.0071 | 0.0000 | 0.0228 |
| Schwersieder | 0.0030 | 0.0000 | 0.0035 | 0.0000 | 0.0000 | 0.0036 | 0.0000 | 0.0114 |

**Tabelle 7b:**

| | Strom-Nr. | | | | | | | |
|---|---|---|---|---|---|---|---|---|
| | **18** | **19** | **21** | **22** | **27** | **28** | **33** | **35** |
| Massenstrom in kg/h | 962 | 12066 | 5419 | 513 | 2068 | 20432 | 29346 | 22503 |
| Konzentrationen in kg/kg | | | | | | | | |
| C4-KWs | 0.0000 | 0.0000 | 0.0000 | 0.0000 | 0.0000 | 0.0000 | 0.0001 | 0.0000 |
| Dimethylether | 0.0000 | 0.0000 | 0.0000 | 0.0000 | 1.0000 | 0.0010 | 0.0000 | 0.0000 |
| Methanol | 0.0000 | 0.0000 | 0.0000 | 0.0000 | 0.0000 | 0.0306 | 0.2079 | 0.0000 |
| Wasser | 0.0000 | 0.0000 | 0.0000 | 0.0000 | 0.0000 | 0.0400 | 0.0000 | 0.0000 |
| Org.Leichtsieder | 0.0000 | 0.0006 | 0.0000 | 0.0000 | 0.0000 | 0.0004 | 0.0000 | 0.0000 |
| 1-Octen | 0.2521 | 0.9850 | 0.0000 | 0.0000 | 0.0000 | 0.5939 | 0.0000 | 0.0000 |
| 3./4-Octen | 0.0462 | 0.0071 | 0.0000 | 0.0000 | 0.0000 | 0.0064 | 0.0000 | 0.0000 |
| 2-Octen | 0.4273 | 0.0073 | 0.0000 | 0.0000 | 0.0000 | 0.0244 | 0.0000 | 0.0000 |
| n-Octan | 0.0023 | 0.0000 | 0.0000 | 0.0000 | 0.0000 | 0.0001 | 0.0057 | 0.0000 |
| Nonene | 0.2510 | 0.0000 | 0.0003 | 0.0000 | 0.0000 | 0.0119 | 0.0000 | 0.0000 |
| Cyclooctan | 0.0168 | 0.0000 | 0.0020 | 0.0000 | 0.0000 | 0.0013 | 0.0000 | 0.0000 |
| 3-Methoxyoctan | 0.0000 | 0.0000 | 0.0000 | 0.0000 | 0.0000 | 0.0000 | 0.0161 | D.0010 |
| 1-Methoxyoctan | 0.0014 | 0.0000 | 0.9700 | 0.0103 | 0.0000 | 0.2576 | 0.7702 | 0.9990 |
| 2-Oetanol | 0.0029 | 0.0000 | 0.0036 | 0.0001 | 0.0000 | 0.0011 | 0.0000 | 0.0000 |
| 1-Oetanol | 0.0001 | 0.0000 | 0.0241 | 0.4941 | 0.0000 | 0.0188 | 0.0000 | 0.0000 |
| C16-KWs | 0.0000 | 0.0000 | 0.0000 | 0.1009 | 0.0000 | 0.0025 | 0.0000 | 0.0000 |
| Dioctylether | 0.0000 | 0.0000 | 0.0000 | 0.2631 | 0.0000 | 0.0066 | 0.0000 | 0.0000 |
| Schwersieder | 0.0000 | 0.0000 | 0.0000 | 0.1316 | 0.0000 | 0.0033 | 0.0000 | 0.0000 |

**Tabelle 7c:**

| | Strom-Nr. | | | | | |
|---|---|---|---|---|---|---|
| | **36** | **38** | **40** | **41** | **43** | **44** |
| Massenstrom in kg/h | 6842 | 13000 | 20241 | 746 | 13778 | 6463 |
| Konzentrationen in kg/kg | | | | | | |
| C4-KWs | 0.0004 | 0.0000 | 0.0001 | 0.0019 | 0.0000 | 0.0002 |
| Dimethylether | 0.0000 | 0.0000 | 0.0000 | 0.0001 | 0.0000 | 0.0001 |
| Methanol | 0.8915 | 0.0470 | 0.3509 | 0.0029 | 0.0469 | 0.9990 |
| Wasser | 0.0000 | 0.9521 | 0.6483 | 0.0039 | 0.9524 | 0.0000 |
| Org. Leichtsieder | 0.0000 | 0.0000 | 0.0000 | 0.0000 | 0.0000 | 0.0000 |
| 1-Octen | 0.0000 | 0.0000 | 0.0000 | 0.0016 | 0.0000 | 0.0000 |
| 3-/4-Octen | 0.0000 | 0.0000 | 0.0000 | 0.0000 | 0.0000 | 0.0000 |
| 2-Octen | 0.0000 | 0.0000 | 0.0000 | 0.0001 | 0.0000 | 0.0000 |
| n-Octan | 0.0243 | 0.0000 | 0.0002 | 0.2174 | 0.0000 | 0.0006 |
| Nonene | 0.0000 | 0.0000 | 0.0000 | 0.0000 | 0.0000 | 0.0000 |
| Cyclooctan | 0.0000 | 0.0000 | 0.0000 | 0.0000 | 0.0000 | 0.0000 |
| 3-Methoxyoctan | 0.0659 | 0.0007 | 0.0004 | 0.6060 | 0.0005 | 0.0000 |
| 1-Methoxyoctan | 0.0179 | 0.0002 | 0.0001 | 0.1653 | 0.0001 | 0.0000 |
| 2-Octanol | 0.0000 | 0.0000 | 0.0000 | 0.0000 | 0.0000 | 0.0000 |
| 1-Octanol | 0.0000 | 0.0000 | 0.0000 | 0.0008 | 0.0000 | 0.0000 |
| C16-KWs | 0.0000 | 0.0000 | 0.0000 | 0.0000 | 0.0000 | 0.0000 |
| Dioctylether | 0.0000 | 0.0000 | 0.0000 | 0.0000 | 0.0000 | 0.0000 |
| Schwersieder | 0.0000 | 0.0000 | 0.0000 | 0.0000 | 0.0000 | 0.0000 |

Durch Zusammenführen der Methanol-haltigen Ströme (4) und (36) in einer gemeinsamen Aufarbeitung können organische Verbindungen über eine einfache Extraktionskolonne abgetrennt werden. Aus der Methanolhaltigen wässrigen Lösung (40) kann dann das Methanol mit 99,9 % Reinheit zurückgewonnen werden.

## Patentansprüche

1. Verfahren zur Herstellung von 1-Octen durch
a) katalytisches Umsetzen eines butadienhaltigen Stroms mit Methanol unter Erhalt eines mindestens 1-Methoxy-2,7-Octadien-halfigen Stroms,
b) katalytische Hydrierung des 1-Methoxy-2,7-Octadien-haltigen Stroms zu einem mindestens 1-Methoxyoctan-haltigen Strom und
c) katalytische Spaltung mindestens eines Teils des 1-Methoxyoctans zu einem Spaltprodukt, enthaltend mindestens Wasser und 1-Octen,
**dadurch gekennzeichnet,**
**dass**
d) das Spaltprodukt aus c) destillativ in eine dampfförmige Leichtsiederfraktion, enthaltend mindestens 1-Octen und Wasser und eine flüssige Schwersiederfraktion, enthaltend mindestens 1-Octen und 1-Methoxyoctan getrennt,
e) die Leichtsiederfraktion ganz oder teilweise kondensiert und in eine wässrige und eine 1-Octen enthaltende, unpolare Phase getrennt,
f) die unpolare Phase aus e) in die Stufe d) rückgeführt und
g) die Schwersiederfiaktion aus d) in eine 1-Octen-haltige und eine 1-Methoxyoctan-haltige Fraktion getrennt wird.

2. Verfahren nach Anspruch 1,
**dadurch gekennzeichnet,**
**dass**
d1) das Spaltprodukt aus c) Dimethylether (DME) enthält und destillativ in eine Leichtsiederfraktion, enthaltend mindestens DME und eine Schwersiederfraktion getrennt und die Schwersiederfraktion zumindest teilweise in die Stufe d) geführt wird.

3. Verfahren nach Anspruch 2,
**dadurch gekennzeichnet,**
**dass** die Schwersiederfraktion aus d1) Methanol aufweist und mit Wasser unter Erhalt eines methanolhaltigen wässrigen und eines unpolaren Stroms gewaschen und der unpolare Strom in die Stufe d) geführt wird.

4. Verfahren nach Anspruch 1,
**dadurch gekennzeichnet,**
**dass** d2) das Spaltprodukt aus c) Methanol aufweist und mit Wasser unter Erhalt eines methanolhaltigen, wässrigen und eines unpolaren Stroms gewaschen wird und dass der unpolare Strom zumindest teilweise in Stufe d) geführt wird.

5. Verfahren nach Anspruch 4,
**dadurch gekennzeichnet,**
**dass** der unpolare Strom mindestens DME enthält und destillativ in eine Leichtsiederfiaktion, enthaltend mindestens DME und eine Schweisiederfraktion getrennt und die Schwersiederfraktion in die Stufe d) geführt wird.

6. Verfahren nach einem der Ansprüche 1 bis 5,
**dadurch gekennzeichnet,**
**dass** in einer Stufe h) die 1-Octen-haltige Fraktion aus g) in eine Fraktion, enthaltend mindestens 1-Octen und eine Fraktion, enthaltend mindestens C₈- und C₉-Olefine getrennt wird.

7. Verfahren nach einem der Ansprüche 1 bis 6,
**dadurch gekennzeichnet,**
**dass** in einer Stufe i) die 1-Methoxyoctan-haltige Fraktion aus g) in eine Leichtsiederfraktion enthaltend 1-Methoxyoctan und eine Schwersiederfraktion, enthaltend mindestens Dioctylether getrennt wird.

8. Verfahren nach Anspruch 7,
**dadurch gekennzeichnet**,
das die Leichtsiederfraktion in Stufe c) zurückgeführt wird.

9. Verfahren nach einem der Ansprüche 1 bis 8,
**dadurch gekennzeichnet,**
**dass** k) der Schritt a) nach der katalytischen Umsetzung eine destillative Abtrennung umfasst, bei der C4-Kohlenwasserstoffe destillativ abgetrennt werden, und der restliche Strom, der einen Gehalt an C4-Kohlenwasserstoffen von kleiner 5 Gew.-% aufweist, in die Stufe b) geführt wird.

10. Verfahren nach einem der Ansprüche 1 bis 9,
**dadurch gekennzeichnet,**
**dass**
1) der Strom aus der Stufe b) destillativ in eine Leichtsiederfiaktion, enthaltend zumindest Methanol, 3-Methoxyoctan und C8-Kohlenwasserstoffe und eine Schwersiederfraktion, enthaltend zumindest 1-Methoxyoctan, getrennt wird, und die Schwersiederfraktion in Stufe c) geführt wird.

11. Verfahren nach einem der Ansprüche 3 oder 4,
**dadurch gekennzeichnet,**
**dass** in einer Stufe o) aus dem wässrigen, methanolhaltigen Strom Methanol und/oder Wasser abgetrennt wird.

12. Verfahren nach Anspruch 11,
**dadurch gekennzeichnet,**
**dass** die wässrige Phase aus e) ebenfalls der Stufe o) zugeführt wird.

13. Verfahren nach Anspruch 11 oder 12,
**dadurch gekennzeichnet,**
**dass** die Leichtsiederfraktion aus 1) ebenfalls der Stufe o) zugeführt wird.

14. Verfahren nach einem der Ansprüche 11 bis 13,
**dadurch gekennzeichnet,**
**dass** aus dem Strom in Stufe o) eine organische Phase abgetrennt und die wässrige Phase destillativ in eine Leichtsiederfraktion, enthaltend Methanol, und eine Schwersiederfraktion, enthaltend Wasser, getrennt wird.

15. Verfahren nach Anspruch 14,
**dadurch gekennzeichnet,**
**dass** die organische Phase durch eine Extraktion abgetrennt wird.

16. Verfahren nach einem der Ansprüche 11 bis 15
**dadurch gekennzeichnet,**
**dass** das Methanol ganz oder teilweise in die Stufe a) (Telomerisation) zurückgeiuhrt wird.

## Claims

1. Process for preparing 1-octene by
a) catalytic reaction of a butadiene-containing stream with methanol to give a stream comprising at least 1-methoxy-2,7-octadiene,
b) catalytic hydrogenation of the 1-methoxy-2,7-octadiene-containing stream to give a stream comprising at least 1-methoxyoctane, and
c) catalytic dissociation of at least part of the 1-methoxyoctane to give a dissociation product comprising at least water and 1-octene,
**characterized in that**
d) the dissociation product from c) is separated by distillation into a gaseous low-boiling fraction comprising at least 1-octene and water and a liquid high-boiling fraction comprising at least 1-octene and 1-methoxyoctane,
e) the low-boiling fraction is completely or partially condensed and separated into an aqueous phase and a 1-octene-containing, nonpolar phase,
f) the nonpolar phase from e) is recirculated to step d) and
g) the high-boiling fraction from d) is separated into a 1-octene-containing fraction and a 1-methoxyoctane-containing fraction.

2. Process according to Claim 1,
**characterized in that**
d1) the dissociation product from c) comprises dimethyl ether (DME) and is separated by distillation into a low-boiling fraction comprising at least DME and a high-boiling fraction which is at least partly passed to step d).

3. Process according to Claim 2,
**characterized in that**
the high-boiling fraction from d1) comprises methanol and is washed with water to give a methanol-containing aqueous stream and a nonpolar stream which is passed to step d).

4. Process according to Claim 1,
**characterized in that**
d2) comprises methanol as dissociation product from c) and is washed with water to give a methanol-containing, aqueous stream and a nonpolar stream which is passed at least partly to step d).

5. Process according to Claim 4,
**characterized in that**
the nonpolar stream comprises at least DME and is separated by distillation into a low-boiling fraction comprising at least DME and a high-boiling fraction which is passed to step d).

6. Process according to any of Claims 1 to 5,
**characterized in that**
the 1-octene-containing fraction from g) is separated in a step h) into a fraction comprising at least 1-octene and a fraction comprising at least C₈- and C₉-olefins.

7. Process according to any of Claims 1 to 6,
**characterized in that**
the 1-methoxyoctane-containing fraction from g) is separated in a step i) into a low-boiling fraction comprising 1-methoxyoctane and a high-boiling fraction comprising at least dioctyl ether.

8. Process according to Claim 7,
**characterized in that**
the low-boiling fraction is recirculated to step c).

9. Process according to any of Claims 1 to 8,
**characterized in that**
k) the step a) comprises, after the catalytic reaction, a distillation step in which C₄-hydrocarbons are separated off by distillation and the remaining stream which has a C₄-hydrocarbon content of less than 5% by weight is passed to step b).

10. Process according to any of Claims 1 to 9,
**characterized in that**
l) the stream from step b) is separated by distillation into a low-boiling fraction comprising at least methanol, 3-methoxyoctane and C₈-hydrocarbons and a high-boiling fraction comprising at least 1-methoxyoctane which is passed to step c).

11. Process according to either of Claims 3 and 4,
**characterized in that**
methanol and/or water is/are separated off from the aqueous, methanol-containing stream in a step o).

12. Process according to Claim 11,
**characterized in that**
the aqueous phase from e) is likewise fed to step o).

13. Process according to Claim 11 or 12,
**characterized in that**
the low-boiling fraction from 1) is likewise fed to step o).

14. Process according to any of Claims 11 to 13,
**characterized in that**
an organic phase is separated off from the stream in step o) and the aqueous phase is separated by distillation into a low-boiling fraction comprising methanol and a high-boiling fraction comprising water.

15. Process according to Claim 14,
**characterized in that**
the organic phase is separated off by extraction.

16. Process according to any of Claims 11 to 15,
**characterized in that**
all or part of the methanol is recirculated to step a) (telomerization).

## Revendications

1. Procédé pour la fabrication de 1-octène par
a) réaction catalytique d'un courant contenant du butadiène avec du méthanol jusqu'à l'obtention d'un courant contenant au moins du 1-méthoxy-2,7-octadiène,
b) hydrogénation catalytique du courant contenant du 1-méthoxy-2,7-octadiène pour l'obtention d'un courant contenant au moins du 1-méthoxyoctane et
c) dissociation catalytique d'au moins une partie du 1-méthoxyoctane pour l'obtention d'un produit de dissociation contenant au moins de l'eau et du 1-octène,
**caractérisé en ce**
**que**
d) le produit de dissociation de c) est séparé par distillation en une fraction à bas point d'ébullition à l'état de vapeur contenant au moins du 1-octène et de l'eau et en une fraction à haut point d'ébullition contenant au moins du 1-octène et du 1-méthoxyoctane,
e) la fraction à bas point d'ébullition est condensée totalement ou partiellement et est séparée en une phase aqueuse et une phase non polaire contenant du 1-octène,
f) la phase non polaire de e) est ramenée à l'étape d) et
g) la fraction à haut point d'ébullition de d) est séparée en une fraction contenant du 1-octène et une fraction contenant du 1-méthoxyoctane.

2. Procédé selon la revendication 1,
**caractérisé en ce**
**que**
d1) le produit de dissociation de c) contient du diméthyléther (DME) et est séparé par distillation en une fraction à bas point d'ébullition contenant au moins du DME et en une fraction à haut point d'ébullition et la fraction à haut point d'ébullition est amenée au moins partiellement à l'étape d).

3. Procédé selon la revendication 2,
**caractérisé en ce**
**que** la fraction à haut point d'ébullition de d1) comprend du méthanol et est lavée avec de l'eau jusqu'à obtention d'un courant non polaire et d'un courant aqueux contenant du méthanol et le courant non polaire est amené à l'étape d).

4. Procédé selon la revendication 1,
**caractérisé en ce**
**que** d2) le produit de dissociation de c) comprend du méthanol et est lavé avec de l'eau jusqu'à obtention d'un courant non polaire et d'un courant aqueux contenant du méthanol et en ce que le courant non polaire est amené au moins partiellement à l'étape d).

5. Procédé selon la revendication 4,
**caractérisé en ce**
**que** le courant non polaire contient au moins du DME et est séparé par distillation en une fraction à bas point d'ébullition contenant au moins du DME et en une fraction à haut point d'ébullition et la fraction à haut point d'ébullition est amenée à l'étape d).

6. Procédé selon l'une des revendications 1 à 5,
**caractérisé en ce**
**que** en une étape h) la fraction de g) contenant du 1-octène est séparée en une fraction contenant au moins du 1-octène et en une fraction contenant au moins des oléfines C₈ et C₉.

7. Procédé selon l'une des revendications 1 à 6,
**caractérisé en ce**
**que** en une étape i) la fraction de g) contenant du 1-méthoxyoctane est séparée en une fraction à bas point d'ébullition, contenant du 1-méthoxyoctane et en une fraction à haut point d'ébullition contenant au moins du dioctyléther.

8. Pocédé selon la revendication 7,
**caractérisé en ce**
**que** la fraction à bas point d'ébullition est ramenée à l'étape c).

9. Procédé selon l'une des revendications 1 à 8,
**caractérisé en ce**
**que** k) l'étape a) comprend une séparation par distillation après la réaction catalytique, pendant laquelle des hydrocarbures C₄ sont séparés par distillation et le courant résiduel qui présente une teneur en hydrocarbures C₄ inférieure à 5 % en poids est amené à l'étape b).

10. Procédé selon l'une des revendications 1 à 9,
**caractérisé en ce**
**que**
l) le courant de l'étape b) est séparé par distillation en une fraction à bas point d'ébullition contenant au moins du méthanol, du 3-méthoxyoctane et des hydrocarbures C₈ et en une fraction à haut point d'ébullition contenant au moins du 1-méthoxyoctane et la fraction à haut point d'ébullition est amenée à l'étape c).

11. Procédé selon l'une des revendications 3 ou 4,
**caractérisé en ce**
**que** en une étape o), on sépare à partir du courant aqueux contenant du méthanol du méthanol et/ou de l'eau.

12. Procédé selon la revendication 11,
**caractérisé en ce**
**que** la phase aqueuse de e) est également amenée à l'étape o).

13. Procédé selon l'une des revendications 11 ou 12,
**caractérisé en ce**
**que** la fraction à bas point d'ébullition de 1) est également amenée à l'étape o).

14. Procédé selon l'une des revendications 11 à 13,
**caractérisé en ce**
**que** à partir du courant à l'étape o) une phase organique est séparée et la phase aqueuse est séparée par distillation en une fraction à bas point d'ébullition contenant du méthanol et en une fraction à haut point d'ébullition contenant de l'eau.

15. Procédé selon la revendication 14,
**caractérisé en ce**
**que** la phase organique est séparée par extraction.

16. Procédé selon l'une des revendications 11 à 15,
**caractérisé en ce**
**que** le méthanol est ramené totalement ou partiellement à l'étape a) (télomérisation).
